# EUROPEAN PATENT SPECIFICATION

(11) **EP 2 417 972 B1**
(45) Date of publication and mention of the grant of the patent: **14.06.2017**
(21) Application number: 10761831.6
(22) Date of filing: 05.04.2010
(51) Int. Cl.: A61K 31/192, A61P 17/02, A61K 31/136

(54) **PHARMACEUTICAL COMPOSITION FOR TREATING OR PREVENTING BURN INJURIES**
PHARMAZEUTISCHE ZUSAMMENSETZUNG ZUR BEHANDLUNG ODER PRÄVENTION VON VERBRENNUNGSVERLETZUNGEN
COMPOSITION PHARMACEUTIQUE DESTINÉE AU TRAITEMENT OU À LA PRÉVENTION DES BRÛLURES

(30) Priority: 06.04.2009 KR 20090029233
(43) Date of publication of application: 15.02.2012
(73) Proprietor: Neurotech Pharmaceuticals Co., Ltd., Suwon-si, Gyeonggi-do 443-270 (KR)
(72) Inventor: GWAG, Byoung-Joo, Giheung-gu Yongin-si, Gyeonggi-do 446-901 (KR); PARK, Ui-Jin, Suwon-si Gyeonggi-do 443-749 (KR)
(74) Representative: Nony
(86) International application number: PCT/KR2010/002070
(87) International publication number: WO 2010/117170

(56) References cited:
- EP-A1- 1 402 888
- EP-A1- 1 674 087
- WO-A1-02/080925
- WO-A1-2004/000786
- WO-A1-2004/014903
- WO-A1-2005/115385
- WO-A2-2005/013885
- US-A- 5 013 540

## Description

### TECHNICAL FIELD

The present invention relates to a pharmaceutical composition and the method of use for treating or preventing skin burn injury in patients or subjects in need.

### BACKGROUND ART

Burns are mainly caused by accidents, and can be classified into thermal burns, burns caused by currents, chemical burns, and radiation burns according to the causes.

The severity of burns can be classified into first-degree, second-degree, third-degree, and fourth-degree burns according to the burned area, depth of burns, the temperature of and the contact time with the object causing burns, and skin condition. In second- or higher-degree burns, scars can be left and hospital cares are required.

First-degree burns make the skin red, and are accompanied by a tingling pain. Also, the outermost layer of the skin layer, the epidermis is damaged and often swollen, accompanied by pain and erythema.

The symptoms disappear within a few days, but light desquamation and pigmentation can be left in its place. After recovery, scars are not left. The case of sun burn is the most common example of first-degree burns.

Second-degree burns affect both the epidermis and dermis, and can cause redness, pain, swelling, and blisters within 24 hours after the accident. Also, this burn also affects the sweat gland and pores. Subjectively, severe burning-sensation or pain can be felt. If blisters burst, an eroded area of the skin is exposed and much of the liquid juice comes out. In case that burned area is over about 15% of body surface area, it needs special attention. Wound heals within a few weeks, but in many cases the pigmentation or depigmentation can be left in its place. If secondary infections occur, local symptoms become more severe and last long.

Third-degree burns affect the epidermis, dermis and hypodermis, make the skin black or translucent white and make blood clot beneath the surface of the skin. These burned areas may be numb, but patients may feel severe pain, and the necrosis of skin tissues and structures require a lot of time for the treatment, remaining scars later. In 2 weeks after the accident, the scab falls off, and ulcer sides appear. There are plenty of secreting fluids and it is easy to bleed, but gradually new tissue formation through epidermis regeneration heals the burn area, remaining scars. If skin necrosis is deep or secondary infection occurs, the healing process is delayed and scar surfaces become irregular, often leading to keloid generation and remaining of transformation or movement disorders. If the burned area is about 10% of body surface area, it needs special attention.

Fourth-degree burns are cases that the burned tissue is carbonized and changed into black, and the layer of fat located under skin layer, ligaments, fascia, muscle or bone also suffers from burns. Fourth-degree burns occur by high-voltage electric injuries and sometimes, in case of fungal infection during deep second- and third-degree burns. If the range of burns is more than 20% of body surface area, body can cause physical reactions, hypotension due to excessive body fluid loss, shock, and acute renal failure may occur and later subsequent wound infection, pneumonia, sepsis, or multiple organ dysfunction syndromes may occur.

For the treatment of burns, it is important to heal the early burn wounds as fast as possible or to reduce the burned area. In the initial burn wound dressings, the initial treatment is emphasized to prevent the transition to deep burns by control of infection and inflammation, maintenance of humid environment, and treatment of growth factors or cytokines helping skin regeneration, local use of heparin etc.

NMDA antagonists have been disclosed in the art for preventing the spread of damage in neuronal tissue that has been selectively burned by surgical laser treatment, as it is described in the US patent No. US 5, 013,540.NMDA antagonists have also been disclosed for preventing primary hyperplasia as well as primary and secondary allodynia (see EP 1 674 087). In WO 2004/000786, this class of compounds are described for preventing and treating acute and chronic neurodegenerative diseases. Also, WO 2005/013885 describes the use of this class of compounds for accelerating wound healing.

If useful therapeutic compounds to treat or prevent burn injury are developed, it would be greatly helpful to treat patients with burns, improve the state, and reduce scars considering the severity of burn injury.

### DISCLOSURE

### Technical Problem

Accordingly, the obj ect of the present invention is to provide a pharmaceutical composition and a medical method using the pomposition useful for treating or preventing skin burn injury.

### Technical Solution

To solve the technical problem, the present invention provides a pharmaceutical composition for treating or preventing skin burn injury, comprising tetrafluorobenzyl derivatives represented by the below chemical formula 1 or its pharmaceutically acceptable salts or solvates as effective agents: 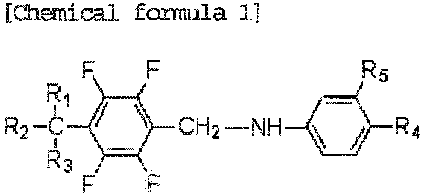 wherein,
R₁, R₂, and R₃ are independently hydrogen or halogen;
R₄ is hydroxy, alkyl, alkoxy, halogen, alkoxy which is substituted with halogen, alkanoyloxy or nitro;
R₅ is carboxylic acid, ester of carboxylic acid with alkyl, carboxyamide, sulfonic acid, halogen, or nitro.

The present invention provides a pharmaceutical composition or a medical method for treating or preventing skin burn injury, comprising tetrafluorobenzyl derivatives represented by the chemical formula 1 or its pharmaceutically acceptable salts or solvates.

Preferably, in the chemical formula 1, alkyl is C₁-C₅ alkyl, and more preferably C₁-C₃ alkyl. More specifically, preferable alkyl includes, but is not limited to, methyl, ethyl, propyl, isopropyl, butyl, sec-butyl and *tert*-butyl. Alkoxy, preferably, is C₁-C₅ alkoxy, and more preferably C₁-C₃ alkoxy. More specifically, preferable alkoxy includes, but is not limited to, methoxy, ethoxy, and propanoxy. Halogen includes, but is not limited to, fluoride, chloride, bromide, and iodide. Preferably, alkanoyl is C₂-C₁₀ alkanoyl, and more preferably C₂-C₅ alkanoyl. More specifically, preferable alkanoyl includes, but is not limited to, ethanoyl, propanoyl, and cyclohexanecarbonyl. Preferably, alkanoyloxy is C₁-C₄ alkanoyloxy.

Preferable examples of the tetrafluorobenzyl derivative represented by the above chemical formula 1 include, but are not limited to, followings:
2-Hydroxy-5-(2,3,5,6-tetrafluoro-4-trifluoromethyl-benzylamino)-benzoic acid (hereinafter, referred to as '2-Hydroxy-TTBA'),
2-Nitro-5-(2,3,5,6-tetrafluoro-4-trifluoromethylbenzylamino) benzoic acid,
2-Chloro-5-(2,3,5,6-tetrafluoro-4-trifluoromethylbenzylamino) benzoic acid,
2-Bromo-5-(2,3,5,6-tetrafluoro-4-trifluoromethylbenzylamino) benzoic acid,
2-Hydroxy-5-(2,3,5,6-tetrafluoro-4-methylbenzylamino)benzoic acid,
2-Methyl-5-(2,3,5,6-tetrafluoro-4-trifluoromethylbenzylamino) benzoic acid,
2-Methoxy-5-(2,3,5,6-tetrafluoro-4-trifluoromethylbenzylamino) benzoic acid,
5-(2,3,5,6-tetrafluoro-4-trifluoromethylbenzylamino)-2-trifluoromethoxy benzoic acid.

In the present invention, skin burns usually refer to the phenomenon that skin cells are destroyed by heat or lead to necrosis. Examples of skin burns include flame burns caused by fire, scalding skin burns caused by hot liquid (water, oil, etc.), contact skin burns caused by contact with hot objects (such as electric irons, rice cookers, etc.), chemical burns caused by strong acids, strong alkalis, sunburns caused by strong ultraviolet light, radiation skin burns caused by exposure to radiation and X-ray, but are not limited to. Also, the invention of burns can be first degree, second degree, third degree, and fourth degree skin burns.

The tetrafluorobenzyl derivative represented by the above chemical formula 1 or its pharmaceutically acceptable salts or solvates can be used for treating or preventing skin burn injury, but are not limited to specific type or degree (severity) of burns.

The tetrafluorobenzyl derivative or its pharmaceutically acceptable salts of the present invention can be prepared by, but is not limited to, the reaction schemes released in US 6,927,303.

Some compounds according to the present invention can be administered in the form of pharmaceutically acceptable salts. The term "pharmaceutically acceptable salts" of the present invention mean salts produced by non-toxic or little toxic base. In case that the compound of the present invention is acidic, base addition salts of the compound of the present invention can be made by reacting the free base of the compound with enough amount of desirable base and adequate inert solvent. Pharmaceutically acceptable base addition salts include, but are not limited to, lithium, sodium, potassium, calcium, ammonium, magnesium or salt made by organic amino. In case that the compound of the present invention is basic, acid addition salts of the compound of the compound can be made by reacting the free base of the compound with enough amount of desirable acid and adequate inert solvent. Pharmaceutically acceptable acid addition salts include, but are not limited to, propionic acid, isobutylic acid, oxalic acid, malic acid, malonic acid, benzoic acid, succinic acid, suberic acid, fumaric acid, mandelic acid, phthalic acid, benzenesulfonic acid, p-tolylsulfonic acid, citric acid, tartaric acid, methanesulfonic acid, hydrochloric acid, bromic acid, nitric acid, carbonic acid, monohydrogencarbonic acid, phosphoric acid, monohydrogen-phosphoric acid, dihydrogen-phosphoric acid, sulfuric acid, monohydrogen-sulfuric acid, hydrogen iodide, and phosphorous acid. In addition, the pharmaceutically acceptable salts of the present invention include, but are not limited to, a salt of amino acid like arginate and an analog of organic acid like glucuronic or galactunoric.

Some of the compounds of the present invention may be hydrated form, and may exist as solvated or unsolvated form. A part of compounds according to the present invention existing as a crystal form or amorphous form, and any physical form is included in the scope of the present invention. In addition, some compounds of the present invention may contain one or more asymmetric carbon atoms or double bonds, and therefore exist in two or more stereoisomeric forms like racemate, enantiomer, diastereomer, geometric isomer, etc. The present invention includes these individual stereoisomers of the compounds of the present invention.

The present invention also describes a pharmaceutical composition comprising the above compound or its pharmaceutically acceptable salts or solvates; and pharmaceutically acceptable excipients or additives. The tetrafluorobenzyl derivative represented by the above chemical formula 1 or its pharmaceutically acceptable salts/solvates of the present invention may be administered alone or with any convenient carrier, diluent, etc. and such a formulation for administration may be single-dose unit or multiple-dose unit.

The pharmaceutical composition of the present invention may be formulated in a solid or liquid form. The solid formulation includes, but is not limited to, a powder, a granule, a tablet, a capsule, a suppository, etc. Also, the solid formulation may further include, but is not limited to, a diluent, a flavoring agent, a binder, a preservative, a disintegrating agent, a lubricant, a filler, etc. The liquid formulation includes, but is not limited to, a solution such as water solution and propylene glycol solution, a suspension, an emulsion, etc., and may be prepared by adding suitable additives such as a coloring agent, a flavoring agent, a stabilizer, a thickener, etc.

For example, a powder can be made by simply mixing the tetrafluorobenzyl derivative of the present invention and pharmaceutically acceptable excipients like lactose, starch, microcrystalline cellulose etc. A granule can be prepared as follows: mixing tetrafluorobenzyl derivatives or its pharmaceutically acceptable salts, a pharmaceutically acceptable diluent and a pharmaceutically acceptable binder such as polyvinylpyrrolidone, hydroxypropylcellulose, etc; and wet-granulating with adequate solvent like water, ethanol, isopropanol, etc, or direct-compressing with compressing power. In addition, a tablet can be made by mixing the granule with a pharmaceutically acceptable lubricant such as magnesium stearate, and tabletting the mixture using a tablet making machine.

The pharmaceutical composition of the present invention may be administered in forms of, but not limited to, oral formulation, injectable formulation (for example, intramuscular, intraperitoneal, intravenous, infusion, subcutaneous, implant), inhalable, intranasal, vaginal, rectal, sublingual, transdermal, topical, etc. depending on the disorders to be treated and the patient's conditions. The composition of the present invention may be formulated in a suitable dosage unit comprising a pharmaceutically acceptable and non-toxic carrier, additive and/or vehicle, which all are generally used in the art, depending on the routes to be administered. A depot type of formulation being able to continuously release drug for desirable time also is included in the scope of the present invention.

The present invention also describes a method of using the tetrafluorobenzyl derivative or its pharmaceutically acceptable salts or solvates for treating and/or preventing skin burn injury; including the administration to objects that require treatment or prevention of skin burn injury with therapeutically effective amount.

For treating skin burn injury, the compound or its pharmaceutically acceptable salts or solvates of the present invention may be administered daily at a dose of approximately 0.01 mg/kg to approximately 1000 mg/kg, preferably approximately 2.5 mg/kg to approximately 500 mg/kg. However, the dosage may be varied according to the patient's conditions (age, sex, body weight, etc.), the severity of patients in need thereof, the used effective compounds, etc. The compounds of the present invention may be administered once a day or several times a day in divided doses, if necessary.

### Advantageous Effects

The present invention relates to a method and a pharmaceutical composition for treating skin burn injury, the compound represented by the chemical formula as an active ingredient or its pharmaceutically acceptable salts.

### DESCRIPTION OF DRAWINGS

Figure 1 shows the protective effect of 2-hydroxy-TTBA against a thermal burn injury using a result of blood chemistry test, that is, through reduction of lactate dehydrogenase in serum
Figure 2 is the photograph showing a result of morphological skin observation at 7 days after a thermal burn injury. This figure shows therapeutic effects of 2-hydroxy-TTBA for the burn injury.
Figure 3 is the photograph showing comparative states of epithelia stained by hematoxylin-eosin according to each experimental groups.
Figure 4 is the photograph showing the state of full-thickness skin in zone of stasis (zone of tissue injury) at 7 days after a thermal burn injury stained by hematoxylin-eosin under 10x microscope.
Figure 5 is the photograph showing the state of live cells of full-thickness skin tissues in zone of stasis (zone of tissue injury) stained by cresyl violet under 10x microscope.
Figure 6 is the photograph showing the state of collagen and muscle fibers of full-thickness skin tissues in zone of stasis (zone of tissue injury) through Masson's trichrom staining.

### MODE FOR INVENTION

Hereinafter, the present invention is described in considerable details to help those skilled in the art understand the present invention. However, various examples according to the present invention can be transformed into other forms, and the scope of the invention should not be construed as being limited to the following examples. Examples of the present invention are provided to explain more completely to the skilled artisan in this field.

### <Example 1> The therapeutic effect against contact burn injury

To confirm the therapeutic effect of 2-hydroxy-TTBA against burn injury, contact burn injury is induced for 30 seconds on the back of rats (on the both side of skin) using a brass comb preheated for 3 minutes in the boiling water (maintaining at 100 °C). After 5 minutes, 2-hydroxy-TTBA 10 mg/5 ml/kg were administered to intravenously for 5 minutes. Since then, twice-a-day administration (at an interval of 10∼12 hours) was sustained for 7 days on the same conditions. The same amount of saline without the compound was administered to the vehicle-treated group in the same manner. Groups of burn experiments were the same as below table 1.

**[Table 1]**

| Groups | Normal group | Burn (control group) | Vehicle-treated group | 2-hydroxy-TTBA-treated group |
|---|---|---|---|---|
| Total number of experimental animals | 6 | 9 | 8 | 7 |
| Number of dead animals | N/A | 2 | 0 | 0 |

| | | | | |
|---|---|---|---|---|
| N/A : not applicable | | | | |

When analyzing the results, number of animals of the normal group for comparison, number of animals in each group, and the number of animals that died within 7 days after the burn injury were shown in above table 1. Two rats in the burn control group died at 5 and 7 days, respectively, after burn injury, but all of another experimental group survived.

### The measurement of lactate dehydrogenase in serum through blood chemistry

Lactate dehydrogenase (LDH) is an enzyme distributed to almost all the tissues and catalyzing reversible reactions between pyruvic acid and lactic acid.

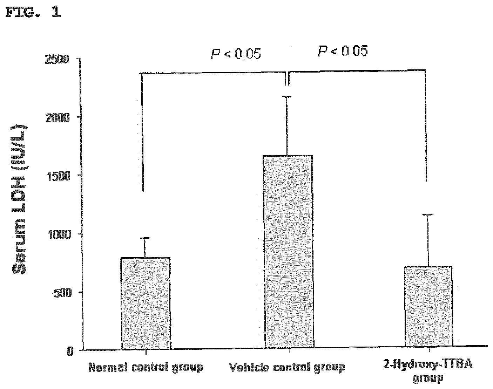

It is known that serum LDH level is elevated when tissues and cells are destroyed. Thus, the amount of LDH was measured in serum samples from each group except hemolyzed samples that may interfere with test results. The result was shown in the figure 1.

As shown in the figure 1, the LDH value of the burn control group was increased approximately 2 times compared to the normal group and the LDH value of 2-hydroxy-TTBA-treated group was reduced significantly compared to burn control group.

### Observation of skin appearance on the back after burn injury

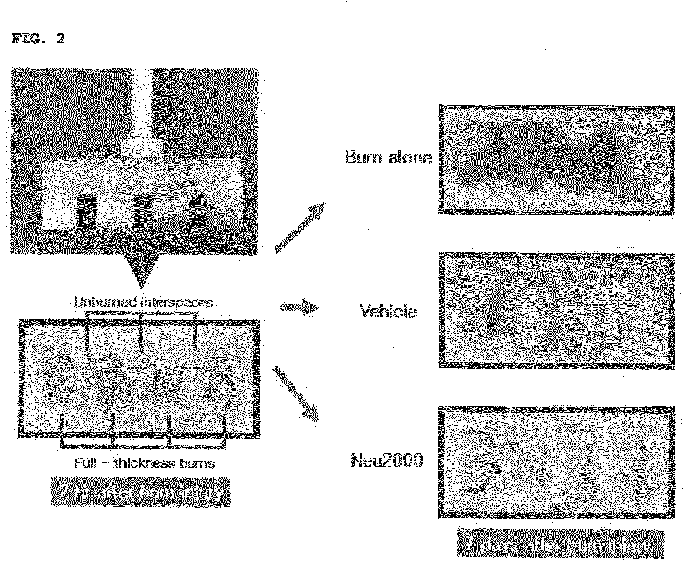

Figure 2 is photographs that observed skin on the back 7 days after burn injury. Full thickness burns were induced in the both sides of back of experimental rats using a preheated brass comb with 4 rectangular shapes of size 10 x 20 mm. At 2 hours after induction, 4 pale-colored or darkish zones of coagulation (or zones of tissue necrosis) and 3 zones of stasis (or zones of tissue injury) appeared on the both sides of back. The zone of coagulation (or zone of tissue necrosis) is a cell region that damaged irreversibly, and the recovery is impossible over time, and the zone of stasis (or zone of tissue injury) is a region that cell necrosis is continued without a specific treatment within 24 ∼ 48 hours, leading to cell death by ischemia caused by continuous fibrin deposition, vasoconstriction, thrombosis, etc.

Therefore, to evaluate the efficacy in this experiment, among the zone of stasis, the remaining 4 regions (a rectangular area indicated the dotted lines) except regions toward the head close to a medication vest among 6 areas appeared in a single rat were analyzed.

As shown in the figure 2, formation of crust in the zone of stasis, switch to the wound, separation of wounds, or elimination of skin may be observed in the burn control group without taking any action.

Formation of eschar such as scab occurred rarely in the vehicle and 2-hydroxy-TTBA-treated group. Specially, the skin of 2-hydroxy-TTBA-treated group was restored to such a good skin condition that hair growth can be observed by the naked eye.

### Histological appearance of eschar formation and wound epidermis formation after burn injury

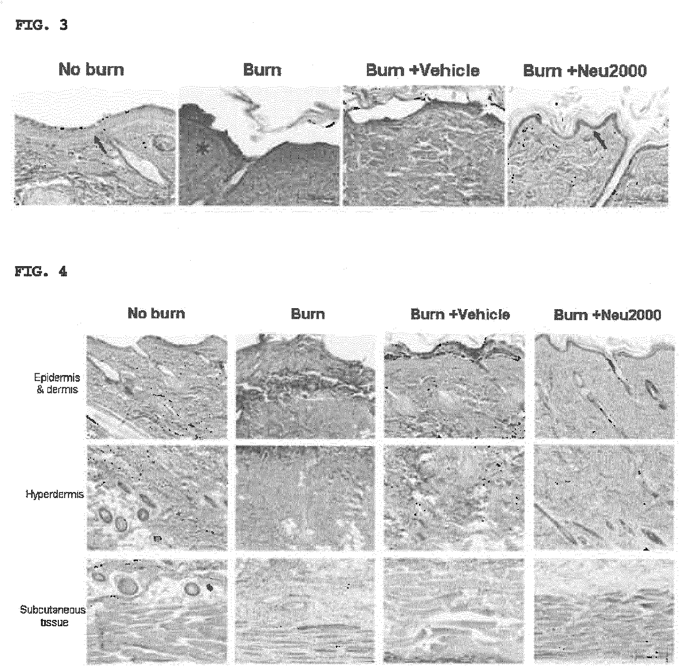

Figure 3 is the result comparing the state of the epithelial layer through hematoxylin-eosin staining of tissues according to the groups. As shown in figure 3, unlike normal controls that normal epithelial layers (part indicated by the arrow) as well as healthy follicles were observed, normal epithelial cells except the inflammatory cells were not observed due to eschar formation in the burn control group. It was identified that wound epidermis formation was in progress in the vehicle-treated and 2-hydroxy-TTBA-treated groups. Some cases that hyperplasia of epidermis thicker than normal epithelial layers occurred could be also observed.

Eschar formation and frequency of wound epidermis formation was measured by analyzing total 28 tissue areas per group (4 zones of stasis per rat, 7 rats per group). As a result, the wound switching frequency of approximately 93% occurred in the burn control group was decreased to about 18% and 4%, respectively, in the veheicle-treated and 2-hydroxy-TTBA-treated groups. Also, the rate of wound epidermis formation was increased by approximately 32% and 72%, respectively, in comparison with the burn control group. The result was shown in the below table 2 (histological appearance : Eschar Formation and frequency of wound epidermis formation).

**[Table 2]**

| Groups | Burn control group | Vehicle-treated group | 2-hydroxy-TTBA-treated group |
|---|---|---|---|
| Eschar formation (%) | 92.857 | 17.857 | 3.571 |
| Wound epidermis formation (%) | 10.714 | 32.143 | 71.429 |

### Histology of full-thickness skin by hematoxylin-eosin staining

Figure 4 was the photograph that observed the state of tissues of full-thickness skin through hematoxylin-eosin staining via 10x microscope.

It showed that epithelium, dermis, subcutaneous tissue and muscle layers have been damaged across the full thickness in the burn control group. It was observed that eschar was formed, the inflammatory cells were infiltrated below it, and there are a large number of inflammatory cells between the subcutaneous tissue and muscle layers. In the vehicle-treated group, schar such as scab was not formed, but a considerable amounts of inflammatory cells were observed all over the skin tissues and subcutaneous tissues, still showing infiltration of the many inflammatory cells below the regenerated epithelium. In 2-hydroxy-TTBA-treated group, infiltration of inflammatory cells was considerably inhibited except in the areas of muscle cells and subcutaneous tissues and not only a wound epidermis formation but also protective effects even in the hair follicles, sebaceous glands and muscle layers appeared.

### Histology of skin full-thickness by cresyl violet staining

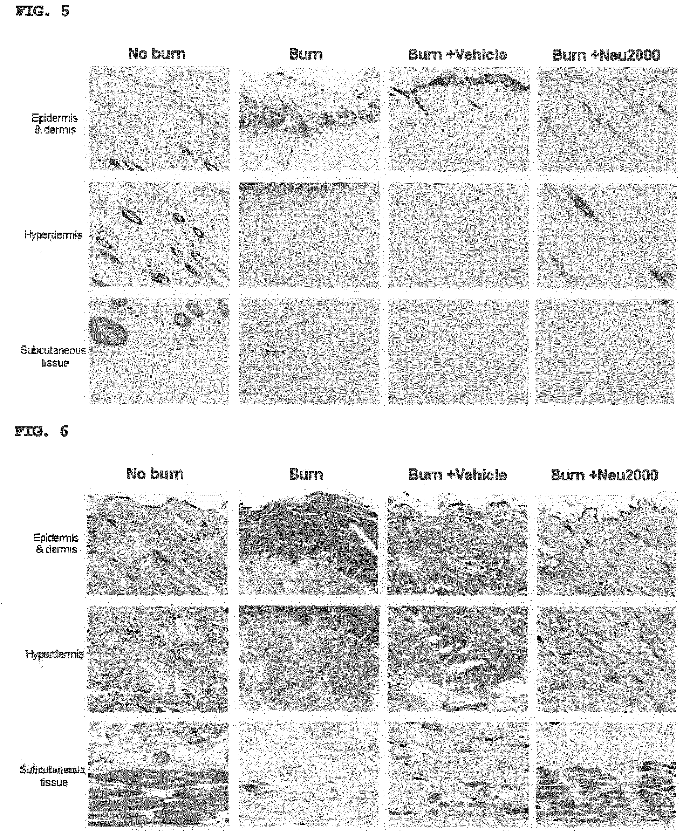

Figure 5 is the photograph observed the live cells in the zone of stasis by cresyl violet staining via 10x microscope. In the burn control group and the vehicle-treated group, follicles were rarely observed and large amounts of inflammatory cells were observed throughout the full-thickness skin. On the other hand, live follicles and epithelia were observed, and relatively few inflammatory cells were observed in 2-hydroxy-TTBA-treated group.

### Histology of full-thickness skin by Masson's trichrom staining

Figure 6 is the photograph that observed collagen of skin tissues and muscle fibers in the zone of stasis by Masson's trichron staining. In the normal group, blue-stained collagen was evenly distributed throughout the dermis, and muscle fibers were stained red. In the burn control group, collagen was deposited irregularly below eschar, the level of staining was weak relative to the normal group. Almost of the muscle fibers were damaged and were not stained. In the vehicle-treated group, the amount of collagen was most abundantly observed relative to the other groups, even to a very high level compared to the normal control. In addition, the muscle fibers with damage were not stained, and significant amount of bleeding and infiltration of inflammatory cells were accompanied. However, in 2-hydroxy-TTBA-treated group, similar to the normal group, epithelium, dermis, subcutaneous fat, and muscles layers were well arranged, showing evenly distributed collages around the live hair follicles, and red-stained muscle fibers.

## Claims

1. The tetrafluorobenzyl derivatives represented by the chemical formula 1 or its pharmaceutically acceptable salts: 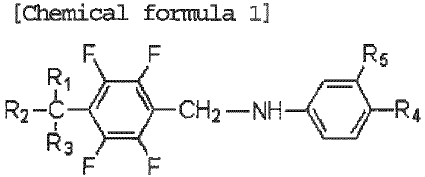 wherein,
R₁, R₂, and R₃ are independently hydrogen or halogen;
R₄ is hydroxy, alkyl, alkoxy, halogen, alkoxy which is substituted with halogen, alkanoyloxy or nitro;
R₅ is carboxylic acid, ester of carboxylic acid with C₁-C₄ alkyl, carboxyamide, sulfonic acid, halogen, or nitro,
for use in a method of treating or preventing skin burn injury.

2. The tetrafluorobenzyl derivatives for use in a method according to claim 1, wherein the tetrafluorobenzyl derivative is any one selected from the group consisting of
2-hydroxy-5-(2,3,5,6-tetrafluoro-4-trifluoromethyl-benzylamino)-benzoic acid,
2-nitro-5-(2,3,5,6-tetrafluoro-4-trifluoromethyl-benzylamino)-benzoic acid,
2-chloro-5-(2,3,5,6-tetrafluoro-4-trifluoromethyl-benzylamino)-benzoic acid,
2-bromo-5-(2,3,5,6-tetrafluoro-4-trifluoromethyl-benzylamino)-benzoic acid,
2-hydroxy-5-(2,3,5,6-tetrafluoro-4-methyl-benzylamino)-benzoic acid,
2-methyl-5-(2,3,5,6-tetrafluoro-4-trifluoromethyl-benzylamino)-benzoic acid,
2-methoxy-5-(2,3,5,6-tetrafluoro-4-trifluoromethyl-benzylamino)-benzoic acid,
5-(2,3,5,6-tetrafluoro-4-trifluoromethyl-benzylamino)-2-trifluoromethoxy benzoic acid,

3. The tetrafluorobenzyl derivatives for use in a method according to claim 2, wherein the tetrafluorobenzyl derivative is 2-hydroxy-5-(2,3,5,6-tetrafluoro-4-trifluoromethyl-benzylamino) benzoic acid.

## Patentansprüche

1. Tetrafluorbenzylderivate der chemischen Formel 1 oder pharmazeutisch unbedenkliche Salze davon: 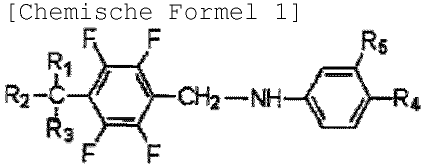 worin
R₁, R₂ und R₃ unabhängig für Wasserstoff oder Halogen stehen;
R₄ für Hydroxy, Alkyl, Alkoxy, Halogen, Alkoxy, das durch Halogen substituiert ist, Alkanoyloxy oder Nitro steht;
R₅ für Carbonsäure, Ester von Carbonsäure mit C₁-C₄-Alkyl, Carboxyamid, Sulfonsäure, Halogen oder Nitro steht,
zur Verwendung bei einer Methode zur Behandlung oder Prävention von Hautverbrennungsverletzungen.

2. Tetrafluorbenzylderivate zur Verwendung bei einer Methode nach Anspruch 1, wobei es sich bei dem Tetrafluorbenzylderivat um eines aus der Gruppe bestehend aus 2-Hydroxy-5-(2,3,5,6-tetrafluor-4-trifluormethyl-benzylamino)benzoesäure, 2-Nitro-5-(2,3,5,6-tetrafluor-4-trifluormethyl-benzylamino)benzoesäure, 2-Chlor-5-(2,3,5,6-tetrafluor-4-trifluormethyl-benzylamino)benzoesäure, 2-Brom-5-(2,3,5,6-tetrafluor-4-trifluormethyl-benzylamino)benzoesäure, 2-Hydroxy-5-(2,3,5,6-tetrafluor-4-methylbenzyl-amino)benzoesäure, 2-Methyl-5-(2,3,5,6-tetrafluor-4-trifluormethyl-benzylamino)benzoesäure, 2-Methoxy-5-(2,3,5,6-tetrafluor-4-trifluormethyl-benzylamino)benzoesäure, 5-(2,3,5,6-tetrafluor-4-trifluormethylbenzyl-amino)-2-trifluormethoxybenzoesäure handelt.

3. Tetrafluorbenzylderivate zur Verwendung bei einer Methode nach Anspruch 2, wobei es sich bei dem Tetrafluorbenzylderivat um 2-Hydroxy-5-(2,3,5,6-tetrafluor-4-trifluormethylbenzylamino)benzoesäure handelt.

## Revendications

1. Dérivés de tétrafluorobenzyle représentés par la formule chimique 1 ou ses sels pharmaceutiquement acceptables : 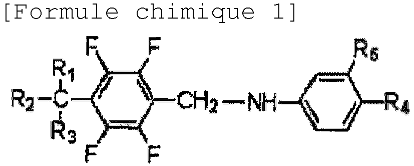 où,
chacun des radicaux R₁, R₂ et R₃ représente indépendamment un atome d'hydrogène ou d'halogène ;
R₄ représente un groupement hydroxy, alkyle, alkoxy, halogène, alkoxy substitué par halogène, alcanoylaxy ou nitro ;
R₅ représente un groupement acide carboxylique, un ester d'acide carboxylique avec un groupement alkyle en C₁-C₄, carboxyamide, acide sulfonique, halogène ou nitro,
pour utilisation dans une méthode de traitement prophylactique ou thérapeutique d'une lésion de type brûlure cutanée.

2. Dérivés de tétrafluorobenzyle pour utilisation dans une méthode selon la revendication 1, où le dérivé de tétrafluorobenzyle est l'un quelconque de ceux choisis dans le groupe constitué par les suivants :
acide 2-hydroxy-5-(2,3,5,6-tétrafluoro-4-trifluorométhyl-benzylamino)-benzoïque,
acide 2-nitro-5-(2,3,5,6-tétrafluoro-4-trifluorométhyl-benzylamino)-benzoïque,
acide 2-chloro-5-(2,3,5,6-tétrafluoro-4-trifluorométhyl-benzylamino)-benzoïque,
acide 2-bromo-5-(2,3,5,6-tétrafluoro-4-trifluorométhyl-benzylamino)-benzoïque,
acide 2-hydroxy-5-(2,3,5,6-tétrafluoro-4-méthyl-benzylamino)-benzoïque,
acide 2-méthyl-5-(2,3,5,6-tétrafluoro-4-trifluorométhyl-benzylamino)-benzoïque,
acide 2-méthoxy-5-(2,3,5,6-tétrafluoro-4-trifluorométhyl-benzylamino)-benzoïque,
acide 5-(2,3,5,6-tétrafluoro-4-trifluorométhyl-benzylamino)-2-trifluorométhoxybenzoïque,

3. Dérivés de tétrafluorobenzyle pour utilisation dans une méthode selon la revendication 2, où le dérivé de tétrafluorobenzyle est l'acide 2-hydroxy-5-(2,3,5,6-tétrafluoro-4-trifluorométhyl-benzylamino)benzoïque.
